# EUROPEAN PATENT SPECIFICATION

(11) **EP 4 044 971 B1**
(45) Date of publication and mention of the grant of the patent: **02.12.2026**
(21) Application number: 20804036.0
(22) Date of filing: 14.10.2020
(51) Int. Cl.: A61F 5/44, A61F 5/442, A61F 5/443, A61F 5/453, A61F 5/455

(54) **CONNECTOR ASSEMBLY INCLUDING CONNECTOR AND PORT**
VERBINDERANORDNUNG MIT VERBINDER UND ANSCHLUSS
ENSEMBLE RACCORD COMPRENANT UN RACCORD ET UN ORIFICE

(30) Priority: 14.10.2019 US 201962914744 P; 13.11.2019 US 201962934836 P; 20.01.2020 US 202062963348 P
(43) Date of publication of application: 24.08.2022
(73) Proprietor: Hollister Incorporated, Libertyville, IL 60048-3781 (US)
(72) Inventor: WINES, James P., Libertyville, IL 60048 (US); HENRY, Jerome A., Libertyville, IL 60048 (US); BOLAND, Patrick, Libertyville, IL 60048 (US); HEALY, Paul, Libertyville, IL 60048 (US); BIGGINS, Damien, Libertyville, IL 60048 (US); FOX, Paul F., Libertyville, IL 60048 (US); CHAMBERS, Mark, Libertyville, IL 60048 (US)
(74) Representative: FRKelly
(86) International application number: PCT/US2020/055545
(87) International publication number: WO 2021/076601

(56) References cited:
- WO-A2-96/37254
- FR-A1- 2 703 593
- US-A- 5 642 744
- US-A1- 2006 025 753

## Description

### Background

### Field of the Disclosure

The present disclosure generally relates to connector assemblies. More particularly, the present disclosure relates to connector assemblies that include a connector and a port.

### Description of Related Art

Several processes for connecting tubing to devices require the use of glue, chemical bonding or welding. The use of glue, chemical bonding and welding can become a bottle neck in the manufacturing process. Additionally, all of these processes are material specific and may not be effective with all of the different possible types of materials that attachable tubes and devices may be made from. Thus, there is a need for improvements in the manner in which devices are connected to one another. US Patent Publication No. 2006/025753 discloses a urinary catheter assembly that can reduce the need for a user to handle the urinary catheter directly. US Patent No. 5,652,744 discloses a cleaning attachment that mates with a standard urostomy collector connector.

### Summary

The invention is set out in the appended set of claims.

### Brief Description of the Drawings

Fig. 1A is a perspective view of an embodiment of a first device connected to a second device;
Fig. 1B is a close-up side view of an embodiment of a first device connected to a second device;
Fig. 2A are elevated cross-sectional views of a connector being connected to a port;
Fig, 2B is a side cross-sectional view of the connector inserted into the port;
Fig. 3A is a perspective view of an embodiment of a connector, showing the connector with a continuous barb;
Fig. 3B is a perspective view of an embodiment of a connector, showing the connector with a barb comprising a plurality of barbs and a spring mechanism;
Fig. 3C is a side view of the connector shown in Fig. 3B;
Fig. 3D is a perspective view of an embodiment of a connector, showing the connector with a recess;
Fig. 3E is a side cross-sectional view of an embodiment of a port, showing the port having a shoulder, a rib, and a tapered lead in;
Fig. 3F is a side cross-sectional view of a connector having a recess and the connector being inserted into an embodiment of a port having a shoulder;
Fig. 3G is a side cross-sectional view of an embodiment of a connector having a recess and the connector being inserted into a port having a bulb-shaped rib and a shoulder;
Fig. 4A is a side view of an embodiment of a bag having a port with an umbrella valve shown in a closed position;
Fig. 4B is a side view of the bag of Fig. 4A, showing the valve in an open position;
Fig. 4C is a side view of an embodiment of a bag having a port with a duckbill valve shown in a closed position;
Fig. 4D is a side view of the bag of Fig. 4C, showing the valve in an open position;
Fig. 5 is a perspective view of an embodiment of a connector having a selectively removable outlet extension;
Fig. 6 is a close-up cross-sectional view of an embodiment of a bag having an integral port;
Fig. 7 is a perspective view of an embodiment of a connector, showing the connector with a bulb-shaped barb;
Fig. 8 is a side cross-sectional view of the connector of Fig. 7 inserted into a port having a recess and a shoulder;
Fig. 9 is a perspective view of an embodiment of a connector, showing the connector with a ball-shaped barb;
Fig. 10 is a side cross-sectional view of the connector of Fig. 9 inserted into a port having a recess and a shoulder;
Fig. 11A is a perspective view of an embodiment of a connector having a recess shown outside of a port having a recess and a double rib;
Fig. 11B is a side cross-sectional view of the connector of Fig. 11A inserted into the port of Fig. 11A;
Fig. 11C is a side cross-sectional view of the connector of Fig. 11A with a modified barb, inserted into the port of Fig. 11A;
Fig. 12 is a side cross-sectional view of an embodiment of a connector having a barb inserted into a port having two ribs and two recesses.

### Description of the Illustrated Embodiments

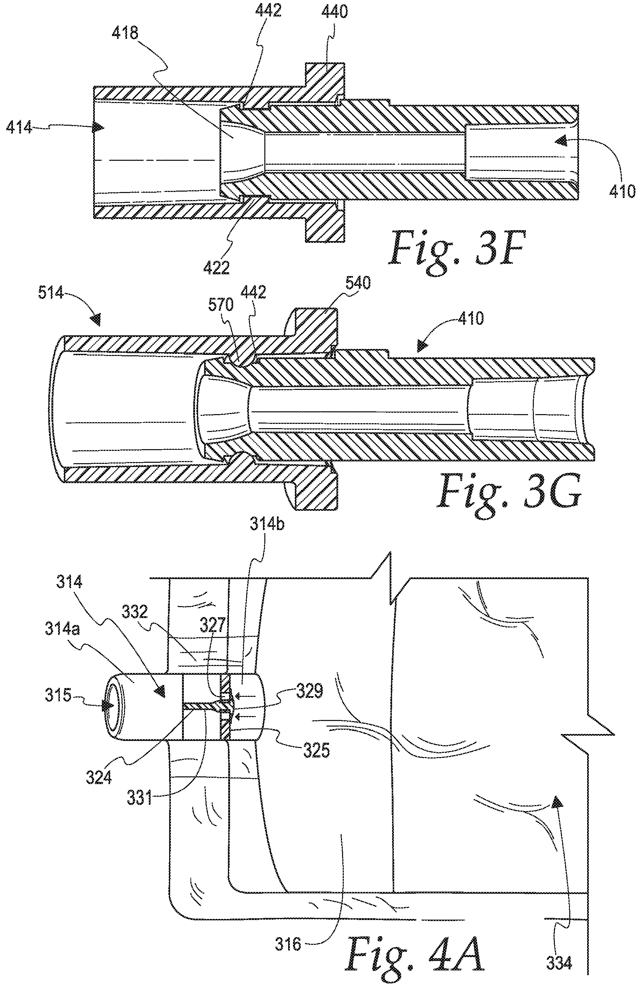

The embodiments disclosed herein are for the purpose of providing a description of the present subject matter, and it is understood that the subject matter may be embodied in various other forms and combinations not shown in detail. Therefore, specific embodiments and features disclosed herein are not to be interpreted as limiting the subject matter as defined in the accompanying claims. The embodiments shown in Figures 3G, 7, 8, 9, and 10 and described below are not according to the invention and are present for illustration purposes only.

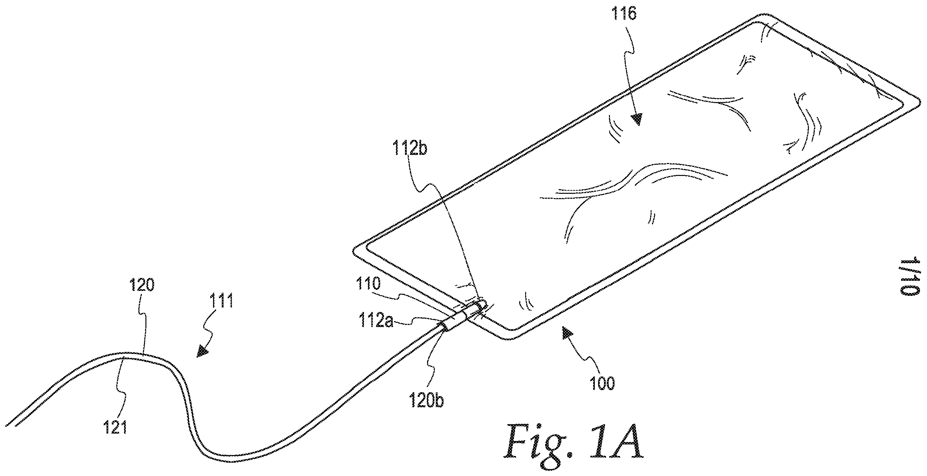

Connector products according to the present disclosure and their individual components may be variously configured without departing from the scope of the present disclosure, but in one embodiment, a connector product is configured as shown in Figure 1A.

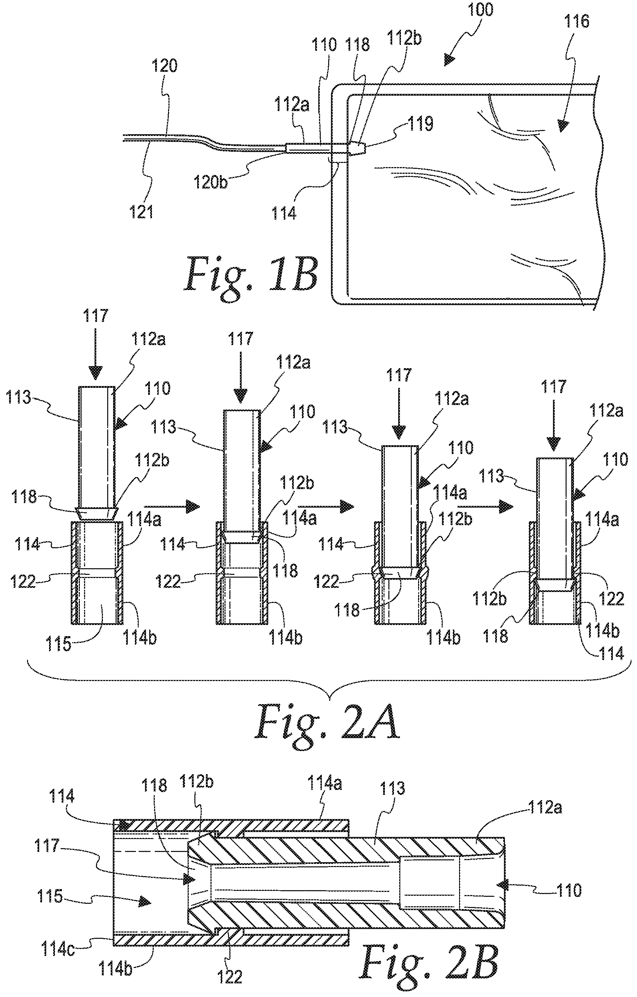

Fig. 1A shows a perspective view of a system 100 that includes two devices connected to one another. The system 100 includes a first device, which may be a conduit 111, and a second device, which may be a bag 116, that are connected to each other. The first device and the second device may be any devices which are connected to each other. For example, the first device and the second device may be of the type wherein fluid flows between the first and second devices. In the illustrated embodiment, the first device is a conduit and the second device is a bag. In other embodiments, one of the devices may be a fluid source or supply or a collection container. The system 100 includes a connector 110 associated with a distal end 120b of a tube 120 of the conduit 111. Referring to Figs. 1B and 2A, the connector product 100 includes a proximal end 112a, a distal end 112b, an outer surface 113 and a lumen 117 extending therethrough. Referring to Figs. 1A and 1B, the proximal end 112a of the connector 110 is configured to be connected to the distal end 120b of the tube 120 so that the lumen 117 (Fig. 2A) of the connector 110 is in fluid communication with a lumen 121 of the tube 120.

Fig. 1B shows a close-up view of the connector product 100. The distal end 112b of the connector 110 defines an opening 119 that is in fluid communication with the bag 116. The distal end 112b also includes at least one barb 118. The bag 116 has a port 114. As shown in Fig. 2A and 2B, the port 114 includes a proximal end 114a, a distal end 114b, and a lumen 115 extending therethrough. The port proximal end 114a is configured to receive the distal end 112b of the connector 110. The port 114 has a rib 122 which is configured to engage the barb 118 of the connector 110 so as to detachably secure the connector 110 to the port 114. The connector lumen 117 is in fluid communication with the bag 116 so that the bag 116 is configured to receive fluid from the connector 110 or fluid may exit the bag 116 through the connector 110. For example, when the bag 116 is a collection container, it receives fluid from the connector 110. When the bag 116 is a supply of fluid, fluid exits the bag 116 through the connector 110.

In one embodiment the port 114 may be a separate component that is attached to the bag 116 and extends through the material of the bag 116. For example, when the bag 116 is formed from a front sheet and a back sheet that are sealed to each about their periphery, the port may be a separate component that extends through a portion of the seal.

In another embodiment the port 114 may be integral with the bag 116, so that the bag 116 and the port 114 are of a single unitary construction made from the same material. For example, in one embodiment the bag 116 may be formed from a first sheet and a second sheet, that are heat sealed to each other about their peripheries. An opening extends through the heat seal and defines the port 114. The portion of the heat seal defining the opening/port is more rigid than at least a second portion of the heat seal. In one embodiment the portion of the heat seal defining the bag opening/port is conditioned to be more rigid that the remaining portion of the heat seal. The opening/port may be conditioned to be more rigid than the remainder of the bag 116 and the remainder of the heat sealed portion by annealing. In alternative embodiments, the opening/port may be conditioned by chemical conditioning, for example by crosslinking.

Fig. 2A shows a close-up view of the connector 110 being inserted into the port 114 (the connector 110-port 114 interface). Fig. 2A shows the connector 110 at various stages of insertion. From left to right, Fig. 2A shows: the connector 110 positioned outside of the port 114; the connector 110 as it is first inserted into the port so that the barb 118 is proximal to the rib 122; the connector inserted such that the barb 118 and rib 122 are aligned; and the connector 110 fully inserted wherein the barb 118 is positioned distal from the rib 122 such that the connector 110 is detachably secured to the port 114.

Fig. 2B shows a cross-sectional view of the connector 110-port 114 interface of Fig. 2A. Fig. 2B shows the connector 110 fully inserted wherein the barb 118 is positioned distal from the rib 122 such that the connector 110 is detachably secured to the port 114. The rib 122 may be positioned anywhere along the port 114. In one embodiment, the rib 122 may be defined by the distal rim 114c of the port 114. When the connector 110 is inserted into the port 114, and the barb 118 and rib 122 are connected, a water tight seal is formed. In one embodiment, the port rib 122 has a diameter that is slightly less than the body of the connector 110, such that the inner wall of the port forms a tight fit to the body of the connector when the connector is inserted into the port. In one embodiment, the port rib 122 will have a diameter smaller than the outer diameter of the body of the connector 110, which may be the portion of the body of the connector 110 that is proximal to the barb 118. In another embodiment, the barb 118 may have an outer diameter that is larger than the inner diameter of the portion of the inner wall of the port 114 that is distal the rib 122. This tight fit creates a liquid-tight seal.

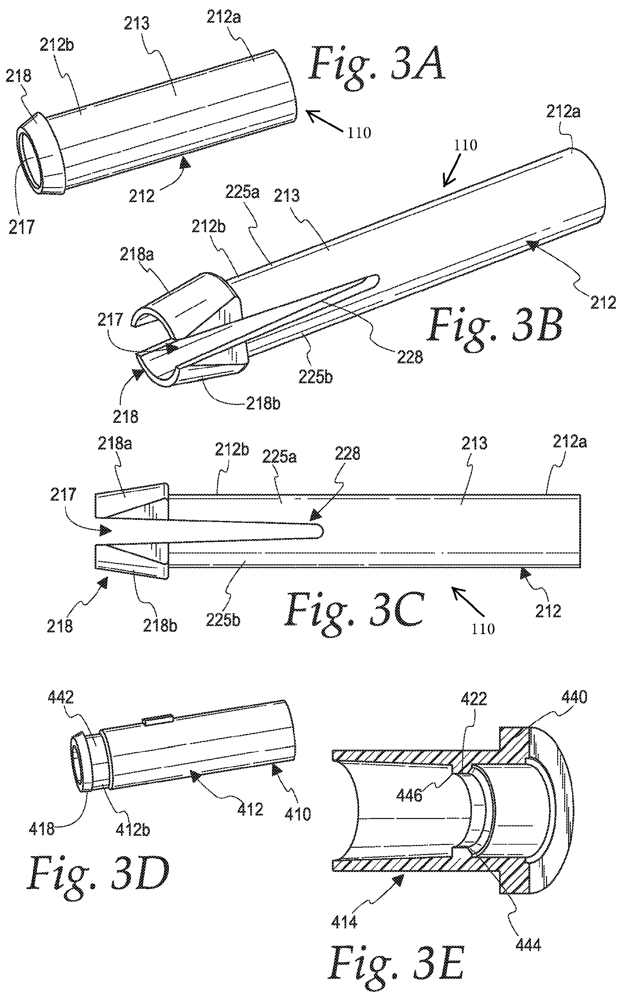

Figs. 3A-3C show various embodiments of the connector which may be employed to connect the first device to the second device, e.g. connect the conduit to the bag. In each of Figs. 3A-3C, the connector 110 comprises a body 212. The body 212 includes a proximal end 212a, a distal end 212b, an outer surface 213, and a lumen extending therethrough 217. The proximal end 212a is configured to be located at a distal end of a tube 120b (as shown in Figs. 1A-1B) so that the lumen of the body 217 is in fluid communication with a drainage lumen 121 of the tube 120. The distal end of the body 212b defines an opening for fluid drainage and includes at least one barb 218 configured for attachment to a bag 116. The at least one barb 218 is configured to engage with the rib 118 of the bag 116 so that the connector 110 is detachably connected to the bag 116. The at least one barb 218 may also be configured to engage the rib 118 when the rib 118 is defined by the port 114.

As shown by Figs. 3A-3C, the barb 218 may extend substantially about the perimeter of the body. In the embodiments shown in Figs. 3A and 3D, the barb 218, 418 is a continuous barb that extends circumferentially around the body 212, 412. In one embodiment, the barb may be a plurality of barbs spaced uniformly or randomly circumferentially about the body.

In the embodiment shown in Figs. 3B and 3C, the barb 218 includes a plurality of barbs: a first barb 218a and a second barb 218b. In the illustrated embodiment, the barbs 218a, 218b are opposed to each other, and may be biased away from one another by a spring mechanism 228. Additionally, as shown in Figs. 3B and 3C, the first barb 218a is located on a first arm 225a of the body 212 and the second barb 218b is located on a second arm 225b of the body 212. The arms 225a, 225b are biased away from one another. In the illustrated embodiment, the arms 225a, 225b are biased away from each other by the elasticity of the material. In other embodiments, the arms may be biased by a separate biasing member such as a spring positioned between the arms.

Though the barb shown in Figs. 3A-3C is substantially circular in shape, other appropriate shapes may be used. For example, if the connector 110 must fit into a square port, the connector may be configured to include a substantially square barb.

In the embodiment shown in Fig. 3D, a connector 410 includes a body 412 having a recess 442 positioned at the body distal end 412b and a distal barb 418. The recess 442 and rib of any of the ports (e.g., 2A, 2B and 3E) disclosed herein may be configured to provide tactile and audible feedback to a user, indicating that the barb 418 is engaged with the rib to secure the connector 410 to the port. The recess 442 and port rib connect in an interference fit which yields a water-tight seal between the connector and the port.

Turning to Fig. 3E, this figure illustrates a port 414 that may be used with any of the suitable connectors disclosed herein. The port 414 is configured to have a rib 422. The rib 422 has two different edge angles. The proximal edge has a tapered lead-in 444. The tapered lead-in 444 may preferably be angled at 30°. However angles between a range of 20° to 60° may be used. The tapered lead-in 444 helps minimize insertion force as the barb passes through the rib 422 during assembly. Furthermore, the distal edge 446 may have a 90° angle to maximize the force required to separate the connector from the port 414, because the proximal edge of the barb may also be angled at 90°. There may be a solid interference fit under a tensile load. Additionally, the port 414 is configured to have a shoulder 440. The shoulder 440 may define a gripping member that may be held during the manufacturing process, or the gripping member may be grasped by the user during use.

For example, during manufacture, the port 414 can be gripped underneath the shoulder 440 by manufacturing equipment. The shoulder 440 provides a gripping point so that the equipment does not have to grip onto a heat seal between a bag and the port 414. Because the equipment grips the shoulder 440 instead of the seal, the shoulder 440 helps minimize the risk that the manufacturing equipment will compromise the integrity of the seal during assembly. Furthermore, if the user connects the conduit to the bag during use, the user may grip the port about the shoulders to hold the port while inserting the connector into the port 414.

Fig. 3F shows a cross-sectional view of an embodiment of the connector 410-port 414 interface where the port 414 includes a shoulder 440 and the connector 410 includes a recess 442. As mentioned above, the recess 442 of the connector 410 and rib 422 of the port 414 may be configured to provide tactile and audible feedback to a user. The barb 418 of the connector 410 is inserted into the port 414 to a suitable depth to engage and be with the rib 422. Fig. 3F shows the connector 410 fully inserted wherein the barb 418 is positioned distal from the rib 422 such that the connector 410 is detachably secured to the port 414. The rib 422 may be positioned anywhere along the port 414. When the connector 410 is inserted into the port 414, and the barb 418 and rib 422 are connected, a water-tight seal is formed. For example, the contact and tolerances between the barb 418 and the rib 422 and/or the rib 422 and the recess 442 may form a water-tight seal. Furthermore, as shown in Fig. 3F, the recess 442 and the rib 422 fit together, strengthening the interference fit between the port 414 and the connector 410. The fit between the recess 442 and the rib 422 may prevent leakage of fluid. In one embodiment, the connector 410-port 414 interface may require a pull-out force of at least 20N to disengage the connector 410 from the port 414.

Fig. 3G shows a cross-sectional view of an embodiment of the connector 410-port 514 interface where the port 514 includes a rib that is bulb-shaped 570. The port 514 also includes a shoulder 540. The connector 410 includes a recess 442. The recess 442 and the bulb-shaped rib 570 engage each other, strengthening the interference fit between the port 514 and the connector 410. In one embodiment, the recess 442 may have a shape that is commensurate with the outer shape of the rib 570. The fit between the recess 442 and the bulb-shaped rib 570 may prevent leakage of fluid. In one embodiment, the connector 410-port 514 interface may require a pull-out force of at least 20N to disengage the connector 410 from the port 514.

Figs. 4A-4D show an embodiment of the bag 316 wherein the bag 316 has a port 314 including a proximal end 314a, a distal end 314b, and a lumen 315 extending therethrough. The port proximal end 314a is configured to receive a connector 110. The port 314 also has a rib where the rib is configured to engage the connector 110, so as to detachably secure the connector 110 to the port 314 and allow the lumen of the port 315 to fluidically communicate with a lumen of the connector 110.

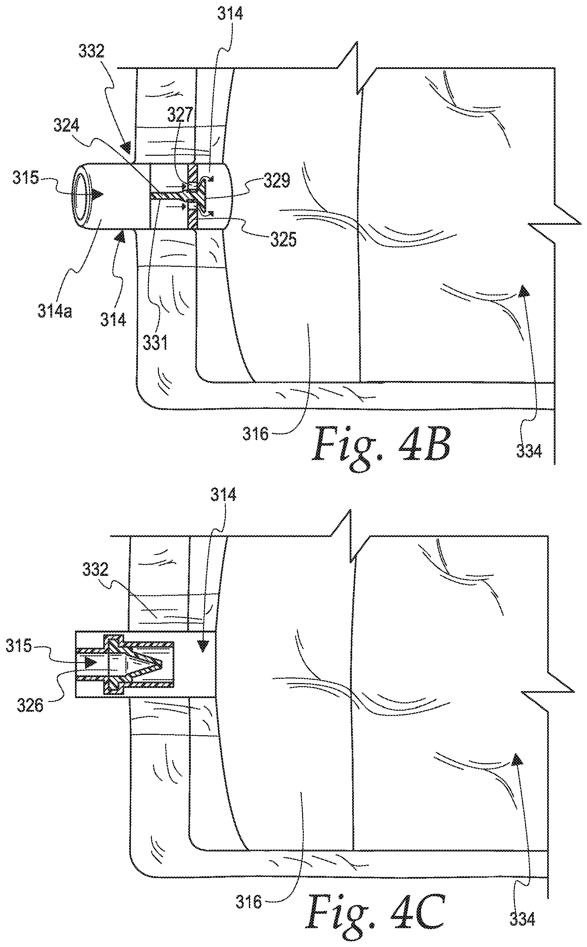

As shown in Figs. 4A and 4B, the port 314 includes a valve 324. The valve 324 is a non-return valve or anti-reflux valve. In the embodiment shown, the valve is an umbrella valve. The valve includes a base 325 extending the width of the port 314. The base 325 includes one or more holes 327 that allow the passage of fluid. The valve also includes a flap 329 that is attached to a stem 331 that is attached to the base 325. The flap 329 is made from an elastic material that flexes away from its original shape to allow fluid flow through the holes 327 and then returns to its original shape to prevent fluid flow through the holes 327. Fig. 4A shows the umbrella valve 324 in a closed position. When the valve 324 is in the closed position, fluid is prevented from flowing or refluxing from the bag 316 back through the port. The arrows in Fig. 4A show that fluid cannot pass through the umbrella valve 324 when it is closed. When the bag 316 includes a supply of fluid, the valve may be reversed such that fluid can exit the bag 316 but cannot enter the bag.

Fig. 4B shows the umbrella valve 324 in an open position. When fluid flows from the conduit through the connector and into the port, the pressure/force of the fluid causes the flap 329 of the valve to flex, thereby uncovering holes 327 and allowing the fluid to flow into the bag 316. The arrows shown in Fig. 4B depict the flow of fluid as it flows through the valve 324 and into the bag 316.

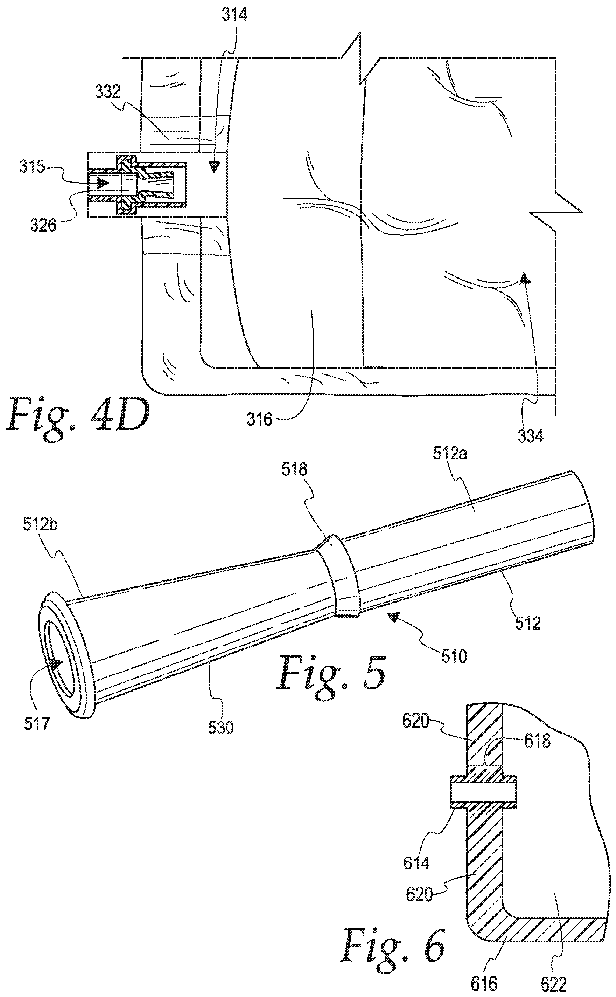

Figs. 4C and 4D show an embodiment of the bag 316 where the port 314 includes a duckbill valve 326. Fig. 4C shows the valve 326 in a closed position. When in the closed position, the duckbill valve 326 prevents fluid from flowing or refluxing from the bag 316 back through the port. Fig. 4D shows the duckbill valve 326 in the open position. When fluid flows from the conduit through the connector and into the port, the pressure/force of the fluid causes the duckbill valve to open allowing fluid to flow into the bag. Though an umbrella valve 324 and a duckbill valve 326 are shown in the drawings, any other appropriate type of valve may be used. Furthermore, a valve may be incorporated into any of the ports disclosed herein. In addition, the valve could be incorporated into the connector instead of the port.

As shown in Figs. 4A-4D in some embodiments the bag 316 may comprise a port section 332 and a container section 334. The port section 332 is configured to include the port 314 and the container section 334 is configured to include the bag's receptacle. In an embodiment, the port section 332 may comprise the portion defining the bag opening. The portion defining the bag opening may be conditioned to be more rigid than at least the container section 334. The port section 332 may be conditioned by annealing. Additionally, the port section 332 may be conditioned by chemical conditioning such as crosslinking. Furthermore, other methods of conditioning to create a more rigid port section 332 may be used. The rigid port section 332 allows for a more stable connection between the connector 110 and the bag 316. The rigidity of the port section 332 helps to stabilize the valves 324,326, allowing them to open and close without rupturing the port 314. In an embodiment a funnel and/or connector 110 may be mechanically bonded to the port section 332 of the bag 316 such that the connector 110 and/or funnel may fluidically communicate with the bag 316.

The embodiment in Fig. 5 shows a connector 510 wherein the body 512 includes a selectively removable outlet extension 530. The extension 530 may be detachably connected adjacent to and distal from the barb 518. The extension 530 includes a lumen which fluidically communicates with the lumen of the body 512 to form a continuous lumen 517. The outlet extension 530 is flared toward its distal end 512b and helps to facilitate controlled fluid drainage. The proximal end 512a is configured to fluidically connect to a conduit from various appropriate devices including, but not limited to, a urinary catheter, or a beverage dispenser. The outlet extension 530 may be used when a user does not need to connect to another device. Alternatively, if the user needs to connect to a device, such as a bag, the user may remove the extension, such as by snapping it off. The user may then connect the connector to the port of the bag, as described above. Additionally, during manufacture, the connectors 510 may be used on both devices that are not used with bags or devices that are connected to bags. That is, in the factory, the connector may be placed on devices that do not require the use of a bag, or the extension could be removed and for example, a device could be connected to a bag prior to distribution to the end user.

Fig. 6 is a close-up cross-sectional view of an embodiment of a bag where the port 614 is integral into the bag. The bag may comprise a first sheet and a second sheet heat sealed about their peripheries wherein the port 614 is defined through the heat seal 616 and the portion 618 of the heat seal 616 defining the port 614 is more rigid than at least a second portion 620 of the heat seal. The port 614 may be conditioned to be more rigid than the remainder of the bag 622. The port 614 may be conditioned by annealing, chemical conditioning such as crosslinking, and/or any other appropriate method for creating a more rigid port 614.

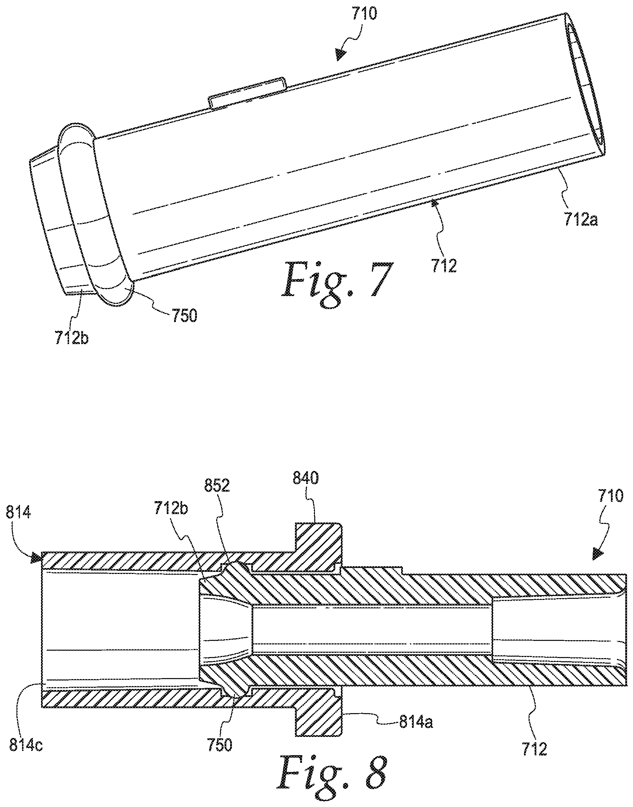

In the embodiment shown in Fig. 7, the connector 710 includes a body 712 having a bulb-shaped barb 750, at or near the body distal end 712b. In the illustrated embodiment, the barb 750 is spaced from the distal end of the body 712. Fig. 8 shows the connector of Fig. 7 inserted into a port 814. In Fig. 8, the port 814 has a recess 852 designed to receive the barb 750. An inner surface 814c of the port 814 may include the recess 852. The recess 852 may be positioned at any point located along the port, and in the illustrated embodiment is positioned near the port proximal end 814a. The barb 750 engages the recess 852 and the barb 750 and recess 852 may be configured to provide tactile and audible feedback to a user, indicating that the barb 750 in engaged with the recess 852 to secure the connector 710 to the port 814. The recess 852 of the port 814 and the bulb-shaped barb 750 of the connector 710 connect in an interference fit which yields a water-tight seal between the connector 710 and the port 814. Additionally, the port 814 is configured to have a shoulder 840. The shoulder 840 may be positioned at the port proximal end 814a.

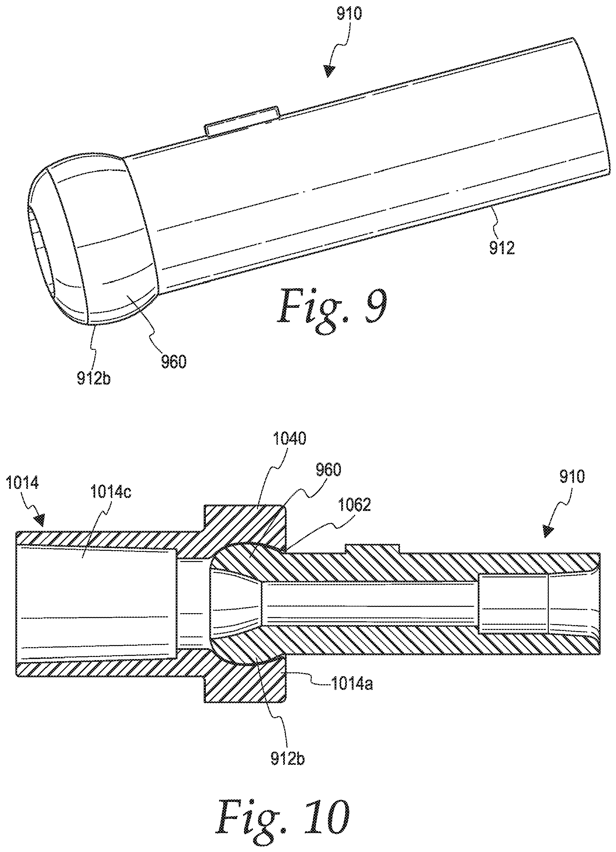

In the embodiment shown in Fig. 9, the connector 910 includes a body 912 having a ball-shaped barb 960, at the body distal end 912b. Fig. 10 shows the connector of Fig. 9 inserted into a port 1014. In Fig. 10, the port 1014 has a recess 1062 designed to receive the ball-shaped barb 960. For example, the recess 1062 may have a hollow ball-shape that receives the barb 960. An inner surface 1014c of the port 1014 may include the recess 1062. The recess 1062 may be positioned at any location along the port, and in Fig. 10 is positioned at the port proximal end 1014a. The recess 1062 may be aligned with the shoulder 1040. The barb 960 fits together with the recess 1062 and the barb 960 and recess 1062 may be configured to provide tactile and audible feedback to a user, indicating that the barb 960 in engaged with the recess 1062 to secure the connector 910 to the port 1014. The recess 1062 and the ball-shaped barb 960 connect in an interference fit which yields a water-tight seal between the connector 910 and the port 1014.

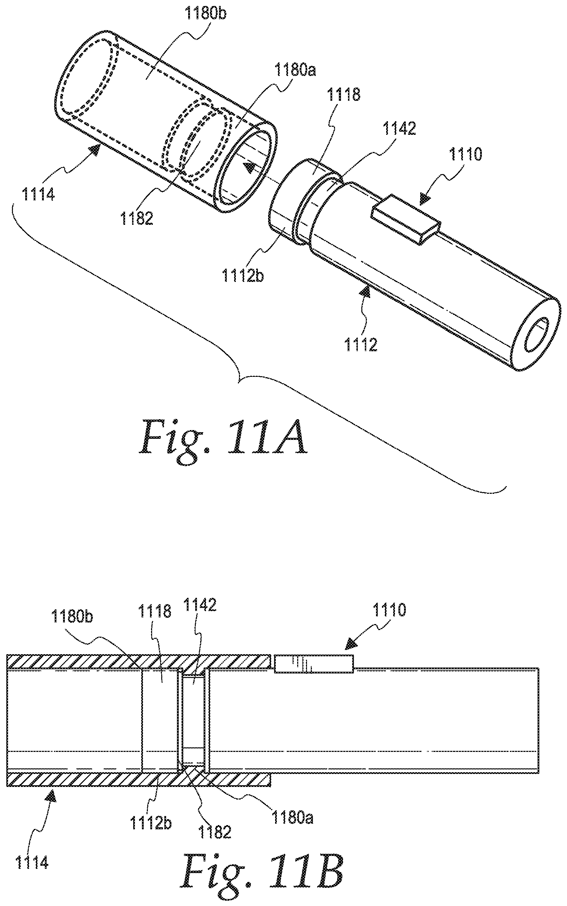

Fig. 11A shows a connector 1110 prior to being inserted into a port 1114. The connector 1110 has a body 1112 that includes a recess 1142 positioned near the body distal end 1112b and a barb 1118, positioned at the body distal end 1112b. The port 1114 has two ribs (a first rib 1180a and a second rib 1180b), and a recess 1182. The recess 1182 and ribs 1180a, 1180b may be configured to provide tactile and audible feedback to a user, indicating that the barb 1118 is engaged with the ribs 1180a, 1180b to secure the connector 1110 to the port 1114. The connector barb 1118 and port recess 1182 connect in an interference fit which yields a water-tight seal between the connector 1110 and the port 1114. The first port rib 1180a and the connector recess 1142 connect in an interference fit while the second port rib 1180b helps hold the distal end of the connector 1112b in place, also in an interference fit. Thus, there are three connection points in this embodiment which yields a strong water-tight seal between the connector 1110 and the port 1114.

Fig. 11B shows the connector 1110 of Fig. 11A inserted into the port 1114 of Fig. 11A. As shown in Fig. 11B the connector barb 1118 is detachably connected to the port recess 1182. The first port rib 1180a is detachably connected to the connector recess 1142, while the second port rib 1180b is detachably secured to the barb 1118 and the distal end of the connector 1112b.

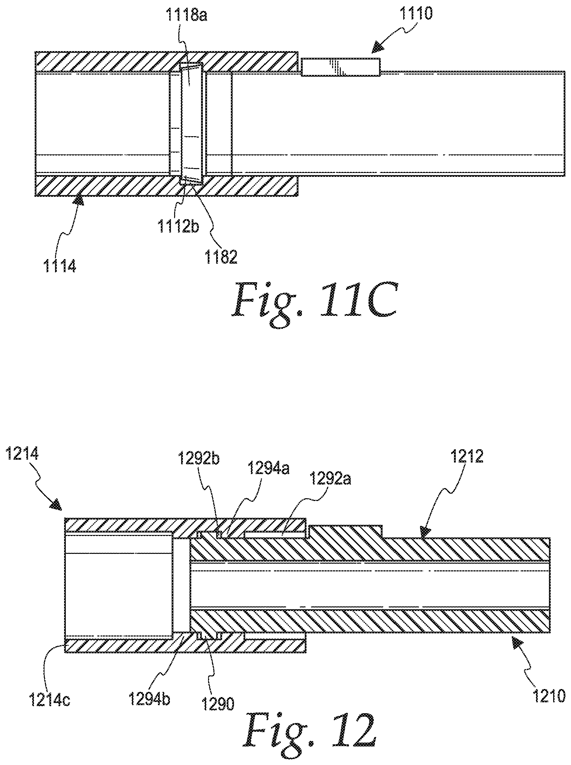

Fig. 11C shows the connector 1110 of Fig. 11A with a modified barb 1118a inserted into the port 1114 of Fig. 11A. The barb 1118a is modified so that it is slightly recessed from the distal end 1112b of the connector 1110. As shown in Fig. 11C the connector barb 1118a is detachably connected with the port recess 1182.

Fig. 12 shows an embodiment of the connector 1210 with a body 1212 having a barb 1290 inserted into a port 1214. The port 1214 has two ribs 1294a, 1294b, a first recess 1292a and a second recess 1292b. The recesses 1292a, 1292b and the ribs 1294a, 1294b may be positioned along the port inner surface 1214c. When the connector 1210 is inserted, the barb 1290 is received by the second recess 1292b. The ribs 1294a, 1294b and the second recess 1292b keep the barb 1290 in place in an interference fit. The interference fit detachably connects the connector 1210 to the port 1214 and yields a water-tight seal between the connector 1210 and the port 1214.

In each of the embodiments described above, the connector may be made from thermoplastic elastomer (TPE) or any other appropriate material. Additionally, the port may be made from Low Density Polyethylene (LDPE) or any other appropriate material. Finally, the bag may be made from a plastic material having an LDPE inner layer, or any other appropriate material.

It will be understood that the structures of the connector and the port could be interchanged. For example, the barb could be on the internal wall of the port and the recess could be on the outer wall of the connector. Additionally, while the port is described as receiving the connector, in alternative embodiments, the connector could receive the port.

## Claims

1. A connector product, comprising:
a tube (120) comprising a tube distal end (120b);
a connector (410) including a connector proximal end, a connector distal end (412b), an outer surface and a connector lumen extending there through, the connector proximal end being connected to the tube distal end (120b), the connector distal end (412b) defining an opening for fluid flow and the connector distal end (412b) including at least one barb (418); and
a bag (116) having a port (414) including a port proximal end, a port distal end, and a port lumen extending therethrough, the port having a rib (422) located distal of the port proximal end, wherein the rib (422) is configured to engage the barb (418) of the connector (410) and the barb (418) and rib (422) are configured so that barb (418) is to be positioned distal of the rib (422) so as to detachably secure the connector (410) to the port (414); and
**characterised in that**
the connector distal end (412b) and the at least one barb (418) being inserted into the port proximal end; and **in that**
the rib including a distal edge (446) having a 90° angle, and the at least one barb (418) including a proximal edge having a 90° angle.

2. The connector product of claim 1 wherein the port (414) is attached and extends into an opening of the bag (116).

3. The connector product of claim 1 wherein the port (414) is integral with the bag (116).

4. The connector product of any one of claims 1-3 wherein at least one of the port (414) or the connector (410) comprises a non-return valve.

5. The connector product of claim 1, wherein the at least one barb (418) extends substantially about a perimeter of the connector.

6. The connector product of claim 5 wherein the at least one barb (418) is a continuous barb that extends about a perimeter of the connector.

## Patentansprüche

1. Verbinderprodukt, umfassend:
einen Schlauch (120), der ein distales Schlauchende (120b) umfasst;
einen Verbinder (410), der ein proximales Verbinderende, ein distales Verbinderende (412b), eine Außenfläche und ein sich hindurch erstreckendes Verbinderlumen aufweist, wobei das proximale Verbinderende mit dem distalen Schlauchende (120b) verbunden ist, wobei das distale Verbinderende (412b) eine Öffnung für Fluidfluss definiert und wobei das distale Verbinderende (412b) mindestens einen Widerhaken (418) aufweist; und
einen Beutel (116) mit einem Anschluss (414), der ein proximales Anschlussende, ein distales Anschlussende und ein sich hindurch erstreckendes Anschlusslumen aufweist, wobei der Anschluss eine Rippe (422) hat, die distal des proximalen Anschlussendes angeordnet ist, wobei die Rippe (422) dazu ausgebildet ist, mit dem Widerhaken (418) des Verbinders (410) in Eingriff zu treten, und wobei der Widerhaken (418) und die Rippe (422) so ausgebildet sind, dass Widerhaken (418) distal der Rippe (422) zu positionieren ist, um den Verbinder (410) lösbar an dem Anschluss (414) zu sichern; und
**dadurch gekennzeichnet, dass**
das distale Verbinderende (412b) und der mindestens eine Widerhaken (418) in das proximale Anschlussende eingeführt; und
dadurch, dass
die Rippe eine distale Kante (446) mit einem Winkel von 90° aufweisend, und der mindestens eine Widerhaken (418) eine proximale Kante mit einem Winkel von 90° aufweisend.

2. Verbinderprodukt nach Anspruch 1, wobei der Anschluss (414) angebracht ist und sich in eine Öffnung des Beutels (116) erstreckt.

3. Verbinderprodukt nach Anspruch 1, wobei der Anschluss (414) einstückig mit dem Beutel (116) ausgebildet ist.

4. Verbinderprodukt nach einem der Ansprüche 1-3, wobei mindestens einer von dem Anschluss (414) oder dem Verbinder (410) ein Rückschlagventil umfasst.

5. Verbinderprodukt nach Anspruch 1, wobei sich der mindestens eine Widerhaken (418) im Wesentlichen um einen Umfang des Verbinders erstreckt.

6. Verbinderprodukt nach Anspruch 5, wobei der mindestens eine Widerhaken (418) ein durchgehender Widerhaken ist, der sich um einen Umfang des Verbinders erstreckt.

## Revendications

1. Produit de raccord, comprenant :
un tube (120) comprenant une extrémité distale de tube (120b) ;
un raccord (410) comportant une extrémité proximale de raccord, une extrémité distale de raccord (412b), une surface extérieure et une lumière de raccord s'étendant à travers celle-ci, l'extrémité proximale de raccord étant reliée à l'extrémité distale de tube (120b), l'extrémité distale de raccord (412b) définissant une ouverture pour l'écoulement de fluide et l'extrémité distale de raccord (412b) comportant au moins une barbe (418) ; et
un sac (116) présentant un orifice (414) comportant une extrémité proximale d'orifice, une extrémité distale d'orifice et une lumière d'orifice s'étendant à travers celui-ci, l'orifice présentant une nervure (422) située de manière distale par rapport à l'extrémité proximale d'orifice, dans lequel la nervure (422) est configurée pour entrer en contact avec la barbe (418) du raccord (410) et la barbe (418) et la nervure (422) sont configurées de sorte que la barbe (418) soit positionnée de manière distale par rapport à la nervure (422) afin de fixer de manière amovible le raccord (410) à l'orifice (414) ; et
**caractérisé en ce que** l'extrémité distale de raccord (412b) et la au moins une barbe (418) sont insérées dans l'extrémité proximale d'orifice ; et **en ce que** la nervure comporte un bord distal (446) présentant un angle de 90°, et la au moins une barbe (418) comporte un bord proximal présentant un angle de 90°.

2. Produit de raccord selon la revendication 1, dans lequel l'orifice (414) est fixé et s'étend dans une ouverture du sac (116).

3. Produit de raccord selon la revendication 1, dans lequel l'orifice (414) est intégré au sac (116).

4. Produit de raccord selon l'une quelconque des revendications 1 à 3, dans lequel au moins l'un de l'orifice (414) ou du raccord (410) comprend un clapet antiretour.

5. Produit de raccord selon la revendication 1, dans lequel la au moins une barbe (418) s'étend sensiblement autour d'un périmètre du raccord.

6. Produit de raccord selon la revendication 5, dans lequel la au moins une barbe (418) est une barbe continue qui s'étend autour d'un périmètre du raccord.
